# EUROPÄISCHE PATENTSCHRIFT

(11) **EP 2 697 231 B1**
(45) Veröffentlichungstag und Bekanntmachung des Hinweises auf die Patenterteilung: **18.02.2015**
(21) Anmeldenummer: 11793424.0
(22) Anmeldetag: 06.12.2011
(51) Int. Cl.: C07C 45/59, C07D 493/04

(54) **KONTINUIERLICH BETREIBBARES VERFAHREN ZUR HERSTELLUNG VON CARBONYLVERBINDUNGEN MITTELS EINES NITROXYLRADIKALHALTIGEN KATALYSATORS**
CONTINUOUSLY USABLE METHOD FOR MANUFACTURING CARBONYL COMPOUNDS USING A NITROXYL RADICAL BASED CATALYST
PROCÉDÉ CONTINU DE FABRICATION DES COMPOSÉS CARBONYLES UTILISANT DES CATALYSATEURS À BASE DE NITROXYL RADICAUX

(30) Priorität: 12.04.2011 EP 11162077
(43) Veröffentlichungstag der Anmeldung: 19.02.2014
(73) Patentinhaber: Evonik Degussa GmbH, 45128 Essen (DE)
(72) Erfinder: KLASOVSKY, Florian, 45721 Haltern am See (DE); HAAS, Thomas, 48161 Münster (DE); TACKE, Thomas, 63755 Alzenau (DE); PFEFFER, Jan Christoph, 63452 Hanau (DE); RIMBACH, Michael, 44649 Herne (DE); VOLLAND, Michael, 48249 Dülmen (DE); JANSSEN, Michiel, 2497 DR Den Haag (NL); SHELDON, Roger, 6997 AZ Hoog-Kappel (NL); HABERLAND, Jürgen, 45721 Haltern am See (DE)
(86) Internationale Anmeldenummer: PCT/EP2011/071911
(87) Internationale Veröffentlichungsnummer: WO 2012/139666

(56) Entgegenhaltungen:
- EP-A1- 1 674 440
- WO-A1-2010/089213
- WO-A1-2010/089223
- US-A- 5 136 103
- US-A- 5 155 279
- US-A- 5 155 280

## Beschreibung

Die vorliegende Erfindung betrifft ein Verfahren zur Oxidation eines primären oder sekundären Alkohols umfassend aliphatische, cycloaliphatische und aromatische Alkohole, bevorzugt zum Aldehyd oder Keton, unter Verwendung einer nitroxylradikalhaltigen Katalysatorzusammensetzung und unter Rückgewinnung dieser Katalysatorenzusammensetzung.

Unter Carbonylverbindungen, die zwei Klassen chemischer Verbindungen, Aldehyde und Ketone, umfassen, finden sich nicht nur eine große Zahl weit verbreiteter Naturstoffe, sondern sie werden auch industriell in erheblichem Umfang sowohl als Edukte als auch als Lösungsmittel bei organischen Synthese eingesetzt. Die Carbonylgruppe stellt eine der wichtigsten funktionellen Gruppen in der organischen Chemie dar.

Im Stand der Technik ist eine große Zahl von Verfahren zur Herstellung von Carbonylverbindungen beschrieben. Dabei werden in der Regel Alkohole als Edukte verwendet, die mit Hinblick auf die Nachhaltigkeit der Carbonylherstellung vorteilhaft sind, weil sie in Form von Kohlenhydraten (Zucker, Saccharide, Stärken, Glycoside, Glykosyle) in großen Mengen in der Natur vorkommen und nach bereits etablierten Verfahren leicht in andere Alkoholderivate (z. B. Anhydrozucker, Zuckeralkohole und dergleichen) umgewandelt werden können.

Die im Stand der Technik beschriebenen Verfahren zur Oxidation von Alkoholen zu Carbonylverbindungen sind mit Nachteilen verbunden, die einer Anwendung im großtechnischen Maßstab entgegenstehen.

So beschreiben Sheldon *et al.* (Sheldon, Roger A.; Arends, Isabel W. C. E. Journal of Molecular Catalysis A: Chemical (2006), 251(1-2), 200-214.) und Minisci et al. (Minisci, Francesco; Punta, Carlo; Recupero, Francesco. Journal of Molecular Catalysis A: Chemical (2006), 251(1-2), 129-149.) ein Verfahren zur Oxidation der Alkohole mittels Sauerstoff unter Verwendung von Nitroxylradikalderivaten sowie unter Zugabe von Übergangsmetallen, welches erfordert, dass die genannten Übergangsmetalle in Form von Salzen nach der Synthese in aufwendiger Weise abgetrennt werden müssen. Diese Salze sind darüber hinaus toxisch.

Andere Verfahren bedienen sich teurer Sauerstoffquellen, z. B. Hypochlorit, Chlorperbenzoesäure, Peroxomonoschwefelsäure, Periodsäure oder Trichloisocyanursäure (z. B. L. Anelli, C. Biffi, F. Montanari, S. Quici, J Org. Chem. 52 (1987) 2559; J.A. Cella, J.A. Kelley, E.F. Kenehan, J. Org. Chem. 40 (1975) 1850; S.D. Rychovsky, R. Vaidyanathan, J. Org. Chem. 64 (1999) 310.; Bolm, Carsten; Magnus, Angelika S.; Hildebrand, Jens P. Organic Letters (2000), 2(8),1173-1175.; S.S. Kim, K. Nehru, Synletter (2002) 616.; De Luca, Lidia; Giacomelli, Giampaolo; Porcheddu, Andrea. Organic Letters (2001), 3(19), 3041-3043.). Zudem enthalten viele der angeführten Reagenzien Halogene, insbesondere Chlor, Brom, Jod, die unter den Reaktionsbedingungen stark korrosiv wirken können und oft zu unerwünschten, die Ausbeute verringernden Nebenreaktionen führen. Gemeinsamer Nachteil der genannten Oxidationsmittel und Hilfsreagenzien ist, dass diese bei der Gewinnung des erwünschten Produktes zu einem erhöhten Trennaufwand führen, weil sie gegebenenfalls in mehreren verschiedenen Verfahrensschritten vom Zielprodukt abgetrennt werden müssen.

Bei einer Reihe von im Stand der Technik beschriebenen Verfahren zur Oxidation von Alkoholen zu Carbonylverbindungen wird als einziger Katalysator oder als Co-Katalysator ein TEMPO (2,2,6,6-Tetramethylpiperidin-1-oxyl)-Derivat eingesetzt. So beschreiben Chinnusamy *et al.* (T. Chinnusamy, O. Reiser, Chem. Sus. Chem. 2010, 3, 1040-1042) die Oxidation von Benzylalkoholen mit Sauerstoff in Gegenwart eines festen TEMPO-Katalysators und in einer Carbonsäure als Lösungsmittel. Nachteilig an dem beschriebenen Verfahren ist, dass wiederum signifikante Mengen von Cobalt- und Mangansalzen eingesetzt werden, die vom Zielprodukt nach Entfernung des festen TEMPO-Derivats in einem zusätzlichen Schritt abgetrennt werden müssen. Die verwendeten TEMPO-Derivate selbst müssen zudem erst aufwändig und kostenintensiv hergestellt werden.

Verschiedene Autoren haben die Verwendung immobilisierter TEMPO-Derivate zur Katalyse der Oxidation von Alkoholen beschrieben ((a) A. Michaud, G. Gingras, M. Morin, F. Béland, R. Ciriminna, D. Avnir, M. Pagliaro, Org. Proc. Res. Dev. 2007, 11, 766-768; (b) A. Michaud, V. Pandarus, L. Tremblay, R. Ciriminna, M. Pagliaro, F. Béland, Top. Catal., 2010, 53, 1110-1113; (c) M. Subhani, M. Beigi, P. Eilbracht, Adv. Synth. Catal. 2008, 350, 2903-290 und (d) C: Roben, A. Studer, W. Hemme, H. Eckert, Synlett 2010, 1110-1114). Der Vorteil eines immobilisierten Katalysators besteht darin, dass er von einer flüssigen Reaktionsmischung leicht abgetrennt werden kann. Die Immobilisierung stellt jedoch neben der Herstellung des Katalysators einen zusätzlichen aufwändigen Prozesschritt dar, der in der Regel mit Verlusten der entsprechenden Verbindung verbunden ist.

WO2010/089223 offenbart ein Verfahren zur Oxidation von Dianhydrohexitolen (Isosorbit) zu 2,6-Dioxabicyclo[3.3.0]octan-4,8-dion durch eine Katalysatorzusammensetzung umfassend ein Nitroxylradikal, eine NO-Quelle, O₂ und eine Carbonsäure. WO2010/089223 offenbart nicht di Recyclierung der Katalysatorzusammensetzung und die Abtrennung des Produktes durch Kristallisation.

WO2010/089213, EP1674440, US5155279, US5155280 und US5136103 enthalten ebenfalls Offenbarungen zur Oxidation von primären oder sekundären Alkoholen in Gegenwart von molekularem Sauerstoff durch eine Katalysatorlösung enthaltend ein Nitroxylradikal, eine NO Quelle und eine Säure.

Vor diesem Hintergrund besteht die der vorliegenden Erfindung zu Grunde liegende Aufgabe darin, ein Verfahren zur Oxidation von Alkoholen zu Carbonylverbindungen mit einem Katalysator zu entwickeln, das ohne die Verwendung toxischer oder aufwändig abzutrennender Salze sowie ohne die Immobilisierung des Katalysators auskommt. Eine weitere der vorliegenden Erfindung zu Grunde liegende Aufgabe besteht darin, Verfahren zur Oxidation von Alkoholen zu Carbonylverbindungen mit einem Katalysator zu entwickeln, bei dem das Verhältnis zwischen Produktausbeute und Menge an eingesetztem Katalysator gegenüber den unter Verwendung der im Stand der Technik beschriebenen Verfahren erzielten Verhältnissen verbessert ist.

Erfindungsgemäß wird die Aufgabe gelöst durch den Gegenstand der beigefügten unabhängigen Ansprüche. Bevorzugte Ausführungsformen ergeben sich aus den Unteransprüchen.

Erfindungsgemäß wird die Aufgabe in einem ersten Aspekt gelöst durch ein Verfahren zur Oxidation eines primären oder sekundären Alkohols umfassend aliphatische, cycloaliphatische und aromatische Alkohole, bevorzugt zu einem Aldehyd bzw. Keton als Kristallinem Reaktionsprodukt, umfassend die folgenden Schritte:
a) Bereitstellen einer Katalysatorzusammensetzung umfassend wenigstens eine nitroxylradikalhaltige Verbindung, wenigstens eine NO-Quelle, wenigstens eine Carbon- oder Mineralsäure oder ein Anhydrid einer Carbon- oder Mineralsäure,
b) Herstellen einer Reaktionsmischung durch Zugabe von wenigstens einem primären oder sekundären Alkohol und einem Sauerstoff umfassenden Gas sowie optional einem oder mehr als einem Lösungsmittel umfasst, zur Katalysatorenzusammensetzung aus Schritt a) bzw. Schritt e),
c) Inkubieren der Reaktionsmischung aus Schritt b) bei einer Temperatur zwischen 0 und 100 °C oder am Siedepunkt des Lösungsmittels,
d) Gleichzeitig mit oder nachfolgend zu Schritt c) Kristallisation des Reaktionsproduktes, und
e) Rückgewinnen der Katalysatorenzusammensetzung durch Entfernung des kristallisierten Reaktionsproduktes aus der in Schritt d) erhaltenen Reaktionsmischung.

In einer ersten Ausführungsform des ersten Aspektes werden die Schritte b) bis e) wenigstens einmal, bevorzugt wenigstens dreimal, wiederholt, und bei einer jeden Wiederholung von Schritt b) wird die Katalysatorenzusammensetzung aus Schritt e) verwendet.

In einer zweiten Ausführungsform des ersten Aspektes, die auch eine Ausführungsform der ersten Ausführungsform darstellt, ist der primäre oder der sekundäre Alkohol ein aliphatischer, cycloaliphatischer oder aromatischer Alkohol.

In einer dritten Ausführungsform des ersten Aspektes, die auch eine Ausführungsform der ersten bis zweiten Ausführungsform darstellt, ist der Alkohol ein mehrwertiger Alkohol.

In einer vierten Ausführungsform des ersten Aspektes, die auch eine Ausführungsform der ersten bis zweiten Ausführungsform darstellt, ist der Alkohol ausgewählt aus der Gruppe, die aliphatische und lineare ω-Hydroxycarbonsäuren, Zuckeralkohole, bevorzugt dizyklische Zuckeralkohole, und Polyole umfasst.

In einer fünften Ausführungsform des ersten Aspektes, die auch eine Ausführungsform der dritten Ausführungsform darstellt, ist der Alkohol aus der Gruppe ausgewählt, die 1,4:3,6-Dianhydro-D-Mannitol (Isomannit), 1,4:3,6-Dianhydro-D-Glucitol (Isosorbit) und 1,4:3,6-Dianhydro-D-Iditol umfasst und ist bevorzugt 1,4:3,6-Dianhydro-D-Glucitol (Isosorbit).

In einer sechsten Ausführungsform des ersten Aspektes, die auch eine Ausführungsform der ersten bis vierten Ausführungsform darstellt, handelt es sich bei der wenigstens einen nitroxylradikalhaltigen Verbindung um eine Verbindung der Formel I oder II, 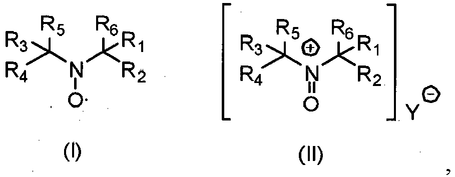 wobei R₁, R₂, R₃, R₄, R₅ und R₆ jeweils unabhängig voneinander aus der Gruppe ausgewählt sind, die (C1-C10)-Alkyl-, (C1-C10)-Alkenyl-, (C1-C10)-Alkoxy-, (C6-C18)-Aryl-, (C7-C19)-Aralkyl-, (C6-C18)-Aryl-(C1-C8)-Alkyl- oder (C3-C18)-Heteroaryl umfasst,
wobei die Substituenten vom Typ R₁, R₂, R₃, R₄, R₅ und R₆ gleich oder verschieden sind und die Substituenten vom Typ R₅ und R₆ zusammen eine (C1-C4)-Alkylenbrücke bilden können, welche gesättigt oder ungesättigt, unsubstituiert oder substituiert sein kann, insbesondere mit einem oder mehreren Substituenten ausgewählt aus der Gruppe umfassend R₁, C1-C8-Amido-, Halogen, Oxy-, Hydroxy-, Amino-, Alkylamino-, Dialkylamino-, Arylamino-, Diarylamino-, Alkylcarbonyloxy-, Arylcarbonyloxy-, Alkylcarbonylamino- und Arylcarbonylaminogruppe,
und wobei Y⁻ ein beliebiges halogenfreies Anion darstellt.

In einer siebten Ausführungsform des ersten Aspektes, die auch eine Ausführungsform der sechsten Ausführungsform darstellt, handelt es sich bei der wenigstens einen nitroxylradikalhaltigen Verbindung um 2,2,6,6-Tetramethylpiperidin-1-oxyl (TEMPO) und/oder die an Position 4 des Heterocyclus substituierten Derivate des 2,2,6,6-Tetramethylpiperidin-1-oxyls,
wobei die Derivate einen oder mehrere Substituenten ausgewählt aus der Gruppe, die R₇, C₁-C₈-Amido-, Halogen, Oxy-, Hydroxy-, Amino-, Alkylamino-, Dialkylamino-, Arylamino-, Diarylamino-, Alkylcarbonyloxy-, Arylcarbonyloxy-, Alkylcarbonylamino- und Arylcarbonylaminogruppen umfasst, aufweisen,
und wobei R₇ aus der Gruppe ausgewählt ist, die (C₁-C₁₀)-Alkyl-, (C₁-C₁₀)-Alkenyl-, (C₁-C₁₀)-Alkoxy-, (C₆-C₁₈)-Aryl-, (C₇-C₁₉)-Aralkyl-, (C₆-C₁₈)-Aryl-(C₁-C₈)-Alkyl- oder (C₃-C₁₈)-Heteroarylgruppe umfasst.

In einer achten Ausführungsform des ersten Aspektes, die auch eine Ausführungsform der ersten bis sechsten Ausführungsform darstellt, ist die NO-Quelle ausgewählt aus der Gruppe, die Sauerstoffsäuren des Stickstoffes sowie deren Salze, bevorzugt Salpetersäure und salpetrige Säure und stickoxidhaltige Gase umfasst.

In einer neunten Ausführungsform des ersten Aspektes, die auch eine Ausführungsform der ersten bis siebten Ausführungsform darstellt, ist die Mineralsäure bzw. ihr Anhydrid ausgewählt aus der Gruppe, die H₂CO₃, H₃PO₄, HNO₃, HNO₂, H₂SO₄, H₂SO₃, H₃BO₃ oder deren Anhydride umfasst.

In einer zehnten Ausführungsform des ersten Aspektes, die auch eine Ausführungsform der ersten bis neunten Ausführungsform darstellt, werden Schritt c) und d) getrennt durchgeführt.

In einer elften Ausführungsform des ersten Aspektes, die auch eine Ausführungsform der ersten bis neunten Ausführungsform darstellt, werden Schritt c) und d) gleichzeitig durchgeführt.

In einer zwölften Ausführungsform des ersten Aspektes, die auch eine Ausführungsform der ersten bis elften Ausführungsform darstellt, wird Schritt c) bei einer Temperatur von unter 70 °C durchgeführt.

In einer dreizehnten Ausführungsform des ersten Aspektes, die auch eine Ausführungsform der zwölften Ausführungsform darstellt, wird Schritt c) bei einer Temperatur von unter 50 °C, bevorzugt unter 45 °C, noch bevorzugter unter 35 °C durchgeführt.

In einer vierzehnten Ausführungsform des ersten Aspektes, die auch eine Ausführungsform der dreizehnten Ausführungsform darstellt, wird Schritt c) bei einer Temperatur von 0 bis 100, bevorzugter 0 bis 70; 10 bis 50; 10 bis 45; noch bevorzugter 10 bis 45; 15 bis 40 °C, 20 bis 40 °C, am bevorzugtesten bei 20 bis 35 °C durchgeführt.

Der vorliegenden Erfindung liegt die überraschende Erkenntnis zu Grunde, dass es möglich ist, eine Katalysatorenzusammensetzung umfassend eine nitroxylradikalhaltige Verbindung, beispielsweise ein TEMPO-Katalysator, nach erfolgtem Reaktionsumsatz durch Entfernung des Reaktionsproduktes mittels Kristallisation aus der Reaktionsmischung in weitgehend aktiver Form zurückzugewinnen und erneut einzusetzen.

In Verfahrensschritt b) wird eine Reaktionslösung hergestellt, die alle Komponenten enthält, die für den Ablauf der Reaktion erforderlich sind. Hierbei wird als Katalysatorenzusammensetzung entweder eine frisch zubereitete Katalysatorenzusammensetzung aus Schritt a) eingesetzt oder eine solche aus Schritt e), die bereits wenigstens einen Reaktionszyklus in Form von Schritt c) katalysiert hat. Darüber hinaus wird in einer bevorzugten Ausführungsform wenigstens ein Edukt hinzugegeben. In einer bevorzugten Ausführungsform umfasst der Begriff "Edukt", wie hierin verwendet, in diesem Zusammenhang jede für die Reaktion notwendige Komponente, die von den Komponenten der Katalysatorenzusammensetzung, bevorzugt der nitroxylradikalhaltigen Verbindung, verschieden ist. In einer besonders bevorzugten Ausführungsform kann auch ein Teil des anfänglich hinzugegebenen Katalysators ersetzt werden, wenn etwa aus Schritt e) noch katalytisch aktive Katalysatorzusammensetzung vorhanden ist, diese zum Zwecke einer höheren Ausbeute aber aufzufüllen ist.

In einer bevorzugten Ausführungsform der vorliegenden Erfindung wird unter dem Begriff "nitroxylradikalhaltige Verbindung", wie hierin verwendet, eine Verbindung verstanden, die die Atomgruppierung 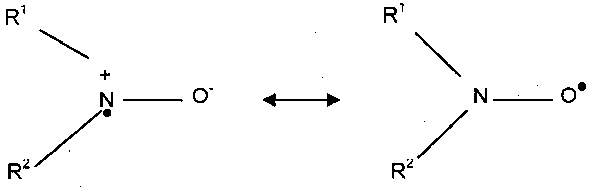 umfasst. Die entsprechenden Nitroxylradikale weisen am α-C-Atom benachbart zum Stickstoff-Atom keine Wasserstoff-Atome auf. In einer bevorzugten Ausführungsform werden die Begriffe "Nitroxylradikal" und "nitroxylradikalthaltige Verbindung", wie hierin verwendet, und dergleichen synonym und austauschbar verwendet und bezeichnen eine Verbindung, die wenigstens ein Nitroxylradikal umfasst.

In dem erfindungsgemäßen Verfahren werden als Nitroxylradikale in der Katalysatorzusammensetzung bevorzugt Verbindungen gemäß der Struktur (I) und/oder salzartige Verbindungen gemäß der der Struktur (II) eingesetzt: 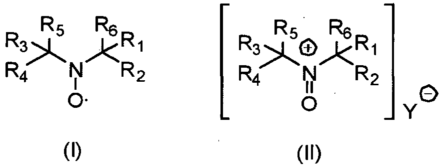 wobei die Substitutenten R₁, R₂, R₃, R₄, R₅ und R₆ unabhängig voneinander aus der Gruppe umfassend (C1-C10)-Alkyl-, (C1-C10)-Alkenyl-, (C1-C10)-Alkoxy-, (C6-C18)-Aryl-, (C7-C19)-Aralkyl-, (C6-C18)-Aryl-(C1-C8)-Alkyl- oder (C3-C18)-Heteroarylgruppe ausgewählt sind, wobei die Substituenten vom Typ R₁, R₂, R₃, R₄, R₅ und R₆ gleich oder verschieden sind und die Substituenten vom Typ R₅ und R₆ zusammen eine (C1-C4)-Alkylenbrücke bilden können, welche gesättigt oder ungesättigt, unsubstituiert oder substituiert sein kann, insbesondere mit einem oder mehreren Substituenten, ausgewählt aus R1, C1-C8-Amido-, Halogen, Oxy-, Hydroxy-, Amino-, Alkylamino-, Dialkylamino-, Arylamino-, Diarylamino-, Alkylcarbonyloxy-, Arylcarbonyloxy-, Alkylcarbonylamino- und Arylcarbonylaminogruppe. In der Struktur (II) ist Y- ein beliebiges halogenfreies Anion.

Es besteht die Möglichkeit, mehr als eine nitroxylradikalhaltige Verbindung einzusetzen.

In einer bevorzugten Ausführungsform werden in dem erfindungsgemäßen Verfahren als Nitroxylradikale 2,2,6,6-Tetramethylpiperidin-1-oxyl (TEMPO) und/oder die an Position 4 des Heterocyclus substituierten Derivate des 2,2,6,6-Tetramethylpiperidin-1-oxyls eingesetzt, wobei die Derivate einen oder mehrere Substituenten ausgewählt aus R₁, C₁-C₈-Amido-, Halogen, Oxy-, Hydroxy-, Amino-, Alkylamino-, Dialkylamino-, Arylamino-, Diarylamino-, Alkylcarbonyloxy-, Arylcarbonyloxy-, Alkylcarbonylamino- und Arylcarbonylaminogruppen aufweisen, worin R1 eine (C₁-C₁₀)-Alkyl-, (C₁-C₁₀)-Alkenyl-, (C₁-C₁₀)-Alkoxy-, (C₆-C₁₈)-Aryl-, (C₇-C₁₉)-Aralkyl-, (C₆-C₁₈)-Aryl-(C₁-C₈)-Alkyl- oder (C₃-C₁₈)-Heteroarylgnxppe bedeutet. Beispiele entsprechender Verbindungen sind 4-Methoxy-2,2,6,6-tetramethylpiperidin-1-oxyl (4-MeO-TEMPO), 4-Oxo-2,2,6,6 tetramethylpiperidin-1-oxyl (4-oxo-TEMPO), 4-Hydroxy-2,2,6,6-tetramethylpiperidin-1-oxyl (4-hydroxy-TEMPO), 4-Benzoyloxy-2,2,6,6-tetramethylpiperidin-1-oxyl (BnO-TEMPO), 4-Acetamido-2,2,6,6-tetramethylpiperidin-1-oxyl, 4-Acetamino-2,2,6,6-tetramethylpiperidin-1-oxyl (AA-TEMPO), 4-Amino-2,2,6,6-tetramethylpiperidin-1-oxyl, *N*,*N*-Dimethylamino-2,2,6,6-tetramethyl-piperidin-1-oxyl (NNDMA-TEMPO), 3,6-Dihydro-2,2,6,6-tetramethyl-1(2H)-pyridinyl-oxyl (DH-TEMPO) oder Bis-(2,2,6,6-tetramethylpiperidin-1-oxyl-4-yl) sebacat, wobei die genannten Beispiele einen oder mehrere Substituenten, ausgewählt aus R₁, C₁-C₈-Amido-, Halogen, Oxy-, Hydroxy-, Amino-, Alkylamino-, Dialkylamino-, Arylamino-, Diarylamino-, Alkylcarbonyloxy-, Arylcarbonyloxy-, Alkylcarbonylamino- und Arylcarbonylaminogruppe, aufweisen können.

In einer bevorzugten Ausführungsform werden in dem erfindungsgemäßen Verfahren die oben genannten Verbindungen AA-TEMPO, 4-Hydroxy-TEMPO, TEMPO und 4-Oxo-TEMPO als Nitroxylradikale eingesetzt. In einer noch bevorzugteren Ausführungsform der vorliegenden Erfindung werden AA-TEMPO, 4-Hydroxy-TEMPO und TEMPO, insbesondere das AA-TEMPO, eingesetzt.

In einer bevorzugten Ausführungsform beträgt der Anteil an Nitroxylradikal mit zunehmender Bevorzugung 0,001 bis 10 mol%, 0,01 bis 5 mol% oder 0,1 bis 2 mol%, bezogen auf die Menge des eingesetzten Alkohols.

Das Verfahren kann zur Oxidation primärer und sekundärer Alkohole eingesetzt werden. Besonders bevorzugt handelt es sich um einen Zuckeralkohol, worunter in einer bevorzugten Ausführungsform, wie hierin, verwendet, ein Kohlenhydrat mit wenigstens einer Hydroxygruppe verstanden wird. In einer besonders bevorzugten Ausführungsform handelt es sich um einen dizyklischen Zuckeralkohol. Unter einem "dizyklischen Zuckeralkohol" wird in einer bevorzugten Ausführungsform, wie hierin verstanden, ein Zuckeralkohol verstanden, der wenigstens transient, zwei Ringsysteme ausbilden kann. In einer bevorzugtesten Ausführungsform handelt es sich bei einem Zuckeralkohol um ein Dianhydrohexitol oder eine Verbindung aus der Gruppe umfassend 1,4:3,6-Dianhydro-D-Mannitol, 1,4:3,6-Dianhydro-D-Glucitol (Isosorbid) und 1,4:3,6-Dianhydro-L-Iditol.

Weiterhin enthält die in dem erfindungsgemäßen Verfahren eingesetzte Katalysatorzusammensetzung mindestens eine NO-Quelle. In einer bevorzugten Ausführungsform der vorliegenden Erfindung können als NO-Quelle Ammoniumnitrat oder -nitrit eingesetzt werden. In einer noch bevorzugteren Ausführungsform können als NO-Quelle stickoxidhaltige Gase wie N₂O, NO, N₂O₃. NO₂, N₂O₄, und N₂O₅ eingesetzt werden. In einer weiteren noch bevorzugteren Ausführungsform finden Sauerstoffsäuren des Stickstoffs als NO-Quelle Verwendung, insbesondere Salpetersäure oder salpetrige Säure. Als NO-Quelle können auch Mischungen der verschiedenen oben genannten NO-Quellen eingesetzt werden. In einer bevorzugten Ausführungsform beträgt der Anteil der eingesetzten NO-Quelle(n) beim erfindungsgemäßen Verfahren 0,001 bis 10 mol-%, bevorzugt von 0,01 bis 5 mol-% und ganz besonders bevorzugt von 0,1 bis 2 mol-%, bezogen auf die Menge an eingesetztem Alkohol. In einer besonders bevorzugten Ausführungsform ist die NO-Quelle mit Sauerstoff regenerierbar und/oder unter Kristallisationsbedingungen abtrennbar. In einer weiteren besonders bevorzugten Ausführungsform weist die NO-Quelle einen Wassergehalt von weniger als 10, bevorzugter weniger als 7,5, 5, 2,5, 1,5, 1 oder 0,5 % (w/v) Wasser auf.

Als Oxidationsmittel wird in dem erfindungsgemäßen Verfahren ein sauerstoffhaltiges Gas eingesetzt. In einer bevorzugten Ausführungsform wird als sauerstoffhaltiges Gas reiner Sauerstoff eingesetzt, es können jedoch auch Mischungen von Sauerstoff mit einem Inertgas oder Luft oder einem an der Reaktion beteiligten Gas eingesetzt werden. Geeignete Inertgase sind beispielsweise Stickstoff, Kohlendioxyd, Helium oder Argon. Als an der Reaktion beteiligte Gase können beispielsweise Stickoxide eingesetzt werden, die bereits vorab bei der Beschreibung der NO-Quellen genannt wurden. Der Partialdruck des Sauerstoffs beträgt bevorzugt 0,1 bis 100 bar, besonders bevorzugt 0,2 bis 50 bar. In einer bevorzugten Ausführungsform ist unter dem Begriff "Sauerstoff umfassendes Gas", wie hierin verwendet, ein Gas oder Gasgemisch zu verstehen, das freien molekularen Sauerstoff, d.h. O₂, umfasst.

Die Oxidationsreaktion kann lösemittelfrei oder unter in Anwesenheit eines Lösemittels erfolgen; bevorzugt ist die Durchführung in einem Lösungsmittel. In einer besonders bevorzugten Ausführungsform der vorliegenden Erfindung wird als Lösungsmittel ein Lösungsmittel verwendet, in dem sich das Alkoholedukt bei einer gegebenen Temperatur besser löst als die während der Oxidation daraus entstehende Carbonylverbindung.

Hierbei werden vorzugsweise polare Lösemittel eingesetzt, insbesondere polare organische Lösungsmittel.

Bevorzugt wird als Lösungsmittel Acetonitril, Tetrahydrofuran, Ethylacetat, Aceton, Diethylether, Methyl-tert.-butylether, tertiäre Alkohole wie tert.-Amlyalkohol, tert.-Butylalkohol, Ester der Kohlensäure wie Dimethylcarbonat, Diethylcarbonat, Kohlenwasserstoffe, Carbonsäuren, Carbonsäureanhydride oder eine Mischung dieser Lösungsmittel eingesetzt. In einer bevorzugten Ausführungsform der vorliegenden Erfindung werden zunehmend bevorzugt 0,1 bis 70 Vol-%, 0,5 bis 60 Vol-% oder 1 bis 50 Vol-% an Lösungsmittel, bezogen auf die eingesetzte Menge an Alkohol, eingesetzt. Besonders bevorzugte Lösemittel umfassen, sind jedoch nicht beschränkt auf die Gruppe der Carbonsäuren bzw. ihrer Anhydride. Als Carbonsäure oder Carbonsäureanhydrid kann in dem erfindungsgemäßen Verfahren beispielsweise Essigsäure, Propionsäure, Buttersäure, Pentansäure, 2-Ethylhexansäure, deren Anhydride oder eine andere Carbonsäure oder ein anderes Anhydrid, das sich in dem Reaktionsgemisch löst, eingesetzt werden. In einer besonders bevorzugten Ausführungsform wird in dem erfindungsgemäßen Verfahren ein aus der Gruppe ausgewähltes Lösungsmittel verwendet, die Essigsäure, Pentansäure und 2-Ethylhexansäure umfasst. Es können auch Mischungen verschiedener geeigneter Carbonsäuren oder Lösungen von Carbonsäuren in einem geeigneten Lösemittel eingesetzt werden. In jedem Fall sollte ein Lösemittel gewählt werden, in dem der Katalysator umfassend das Nitroxylradikal ausreichend löslich ist. Der Fachmann ist in der Lage, die Löslichkeit einer Substanz wie der des Katalysators in einem Lösungsmittel im Rahmen routinemäßigen Experimentierens zu bestimmen und festzustellen, welche Menge das Lösungsmittel aufnehmen kann, ohne dass es zur Präzipitation des Katalysators oder ähnlichen unerwünschten Effekten kommt.

In einer bevorzugten Ausführungsform der vorliegenden Erfindung wird Schritt c) bei einer Temperatur T_{O} von 0 bis 100 °C, oder am Siedepunkt des Lösungsmittels durchgeführt. Besonders bevorzugt ist 10 °C < T_{O} < 80 °C und ganz besonders bevorzugt 15 °C < T_{O} < 50 °C

Der Gesamtdruck bei der Oxidation im erfindungsgemäßen Verfahren beträgt vorzugsweise 1 bis 300 bar, bevorzugt 1 bis 50 bar und ganz besonders bevorzugt 1 bis 5 bar.

Der Verfahrensschritt c) kann sowohl als Batch-, Semi-Batch- oder in kontinuierlicher Fahrweise durchgeführt werden. Des Weiteren ist der Verfahrensschritt c) an keinen bestimmten Reaktortyp gebunden, vielmehr kann der Verfahrensschritt in einem Rührkessel, in einem Rohrreaktor, in einer Kesselkaskade oder einer Kombination dieser Reaktortypen durchgeführt werden. In einer Ausführungsform der vorliegenden Erfindung wird zunächst der Alkohol in einem geeigneten Lösungsmittel gelöst oder suspendiert, anschließend wird die Katalysatorzusammensetzung dieser Lösung oder Suspension einzeln oder als Mischung zugegeben. Anschließend werden Druck und Temperatur eingestellt. Es ist jedoch auch möglich, die Katalysatorzusammensetzung vorzulegen und die Lösung oder Suspension des Alkohols der Katalysatorzusammensetzung zuzugeben. Im Falle einer kontinuierlichen Verfahrensführung wird vorzugsweise der Alkohol mit den Reaktionsgasen in der Ausführung eines Rieselbettes oder einer Blasensäule zugeführt. Besonders vorteilhaft in dem erfindungsgemäßen Verfahren ist es, wenn der Gehalt an Wasser in der Reaktionszusammensetzung möglichst gering ist, da hohe Anteile an Wasser die Ausbeuten verringern können. Darüber hinaus kann die Reaktion durch die Entfernung des Reaktionswassers aus dem Reaktionsgeschehen beschleunigt werden.

Um den Wasseranteil möglichst gering zu halten, bieten sich mehrere Verfahrensvarianten an. Im Sinne einer effizienten Prozessführung unter Vermeidung zusätzlicher Koppelprodukte sollte auf die Zugabe von dem Reaktionsgemisch auf chemischem oder physikalischem Wege Wasser entziehenden Hilfssubstanzen verzichtet werden. Stattdessen kann freiwerdendes Wasser in Verfahrensschritt c) kontinuierlich aus der Reaktionsmischung abdestilliert werden. Eine bevorzugte Verfahrensvariante besteht darin, das Reaktionswasser chemisch an die zu erzeugende Carbonylverbindung zu binden, wobei diese Bildung der Carbonylhydrate freiwillig verläuft. Nach Isolierung der Carbonylhydrate gemäß dem nachfolgend ausgeführten Verfahrensschritt d) können diese Carbonylhydrate in einem optionalen Verfahrensschritt wieder in Wasser und Carbonylverbindung aufgetrennt werden. Ein solcher Verfahrensschritt kann beispielsweise eine Trocknung oder Vakuum-Sublimation umfassen. Stellen die in dieser Erfindung beschriebenen Carbonylverbindungen jedoch Ausgangsprodukte für Folgesynthesen dar, können alternativ auch die Carbonylhydrate in unveränderter Form, d.h. ohne vorherige Wasserabspaltung, eingesetzt werden. Solche Folgesynthesen können beispielsweise Kondensationsreaktionen der Carbonylgruppe, wie die Reaktionen mit Nukleophilen, z.B. Ammoniak, Amine, Hydrazine und dergleichen, umfassen.

Die Kristallisation der Carbonylverbindung bzw. des Carbonylhydrats in Schritt d) kann je nach Löslichkeit von Alkohol und Carbonylverbindung bzw. Carbonylhydrat sowie der eingesetzten Eduktkonzentration sequentiell nach Durchführung der Oxidationsreaktion oder auch gleichzeitig mit dieser in Verfahrensschritt c) erfolgen. Im sequentiellen Betrieb erfolgen Oxidation und Kristallisation bevorzugt absatzweise; jedoch ist auch jeweils eine kontinuierliche Fahrweise möglich, wenn die Orte der Reaktion und der Kristallisation räumlich, z. B. durch nacheinandergeschaltete Apparate, getrennt sind. Die genauen Bedingungen, unter denen die Carbonylverbindungen zur Kristallisation gebracht werden kann, kann der Fachmann im Rahmen routinemäßigen Experimtentierens ermitteln. Geeignete Vorgehensweisen und zu beachtende Parameter sind im Stand der Technik beschrieben, beispielsweise in "Organikum - Organisch-chemisches Grundlagen-praktikum", 22. Auflage, Wiley-VCH-Verlag.

Schritt e) des erfindungsgemäßen Verfahrens umfasst das Rückgewinnen der Katalysatorenzusammensetzung durch Entfernung des kristallisierten Reaktionsproduktes aus der in Schritt d) erhaltenen Reaktionsmischung. Dem Fachmann sind einfach durchzuführende geeignete Verfahren zur Entfernung eines kristallisierten Reaktionsproduktes aus einer flüssigen Reaktionslösung bekannt. Beispielsweise kann das Gemisch zentrifugiert oder einfach stehen gelassen werden, bis sich die unlöslichen Kristalle am Boden des Gefäßes abgesetzt haben, woraufhin die wässrige Lösung einfach abdekantiert wird. Alternativ kann die Lösung filtriert werden. Die Entfernung des kristallisierten Reaktionsproduktes kann auch die Entfernung von damit verbundenem Wasser umfassen. Die genannten Trennoperationen können auch kontinuierlich durchgeführt werden.

Im Falle der sequentiellen Kristallisation kann sich die Kristallisationstemperatur T_{K} in Verfahrensschritt d) von der Oxidationstemperatur T_{O} in Verfahrensschritt c) unterscheiden. Dabei muss T_{K} so gewählt werden, dass unter den gegebenen Konzentrationsbedingungen weder die Löslichkeitsgrenze des ggf. noch in der Reaktionsmischung verbliebenen Alkoholeduktes noch die Löslichkeitsgrenze des in der Reaktionsmischung enthaltenen Katalysatorsystems unterschritten wird. In einer bevorzugten Ausführungsform erfolgt die Kristallisation durch Absenkung der Temperatur um wenigstens 10 °C und/oder auf eine Temperatur zwischen 0 und 25, bevorzugt 0 und 20, noch bevorzugter 5 und 18, am bevorzugtesten 10 bis 17,5 °C.

Im Falle der parallelen Kristallisation ist T_{K} = T_{O}, wobei die Löslichkeit der Carbonylverbindung bzw. des Carbonylhydrats somit bei T_{O} deutlich geringer ist als jene des in der Reaktionsmischung befindlichen Edukts, so dass die Carbonylverbindung bzw. das Carbonylhydrat während der Oxidation ausfällt. In diesem Fall ist eine kontinuierliche Fahrweise bevorzugt, bei der die Reaktionsmischung permanent durch eine ggf. räumlich vom Reaktor getrennten Trennungsapparat (z.B. eine Filterpresse) geführt und die resultierende, an Carbonylverbindung bzw. Carbonylhydrat abgereicherte Reaktionsmischung wieder in den Reaktor zurückgeleitet wird.

Bevorzugt ist das Verfahren der parallelen Kristallisation im Rahmen einer kontinuierlichen Betriebsweise von Verfahrensschritt c).

Das aus dem beschriebenen Verfahrensschritt e) erhaltene, ggf. carbonylhydrathaltige Produkt wird in einer bevorzugten Ausführungsform in einem optionalen Verfahrensschritt durch Wasserentfernung in die wasserfreie Carbonylverbindung überführt.

Die Temperatur während der Reaktion in Schritt c) muss einerseits der optimalen Ausbeute und dem Erfordernis eines raschen Reaktionsablaufes, andererseits der Haltbarkeit und Stabilität der Reagenzien und Produkte Rechnung tragen. In einer bevorzugten Ausführungsform der vorliegenden Erfindung wird Schritt c) mit zunehmender Bevorzugung bei einer Temperatur von unter 70, 65, 60, 55, 50, 45, 40 oder 30 °C ausgeführt. In einer bevorzugten Ausführungsform bedeutet der Begriff "bei einer Temperatur von unter X °C ausgeführt", wie hierin verwendet, dass die über den Zeitverlauf von Schritt c) gemittelte Temperatur unter X °C liegt.

Die vorliegende Erfindung wird weiter anhand der folgenden Figur und des folgenden Beispiels erklärt, aus denen sich weitere Merkmale, Ausführungsformen und Vorteile ergeben.

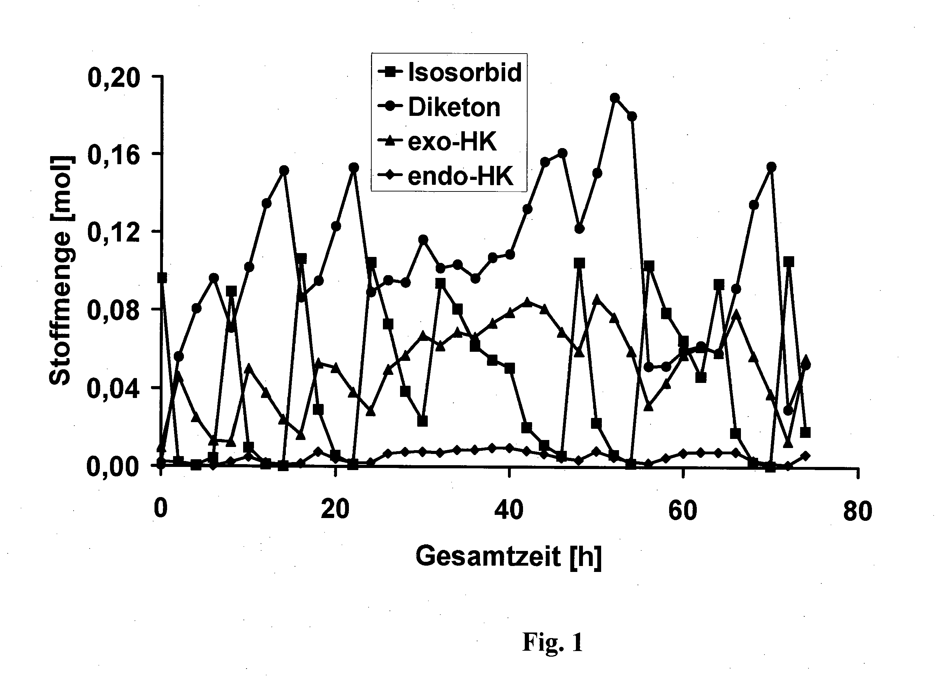

Fig. 1 zeigt die Stoffmengen von Edukt, Produkt und Zwischenprodukten bei Oxidation von Isosorbid in einem Experiment mit 9-facher Wiedergewinnung des TEMPO-Katalysators.

### Beispiel 1: Oxidation von Isosorbid

In einem im Wasserbad beheizten 250 mL-Vierhalskolben mit Magnetrührer, Rückflußkühler, Innenthermometer und Gaseinleitungsrohr (mit Fritte) werden unter Rühren 14.63 g Isosorbid (100 mmol) in 78.94 g Eisessig aufgelöst. Anschließend wird die Reaktionsmischung innerhalb einer Zeit von 20 min mit Sauerstoff gesättigt. Währenddessen werden 1.5 g AA-TEMPO (7 mmol) in 25.03 g Eisessig aufgelöst. Nach Ende der Begasungszeit wird diese Lösung zusammen mit 0.47 g rauchender Salpetersäure (7 mmol) zur Reaktionsmischung hinzugefügt und die Reaktion damit gestartet. Nach 6 h Rühren bei 25 °C wird die Reaktionsmischung auf 15 °C abgekühlt und 12 h kristallisiert. Ausfallendes Diketon wird danach abgesaugt und die resultierende Mutterlauge wieder mit soviel Isosorbid, AA-TEMPO, Salpetersäure und Essigsäure versetzt, dass die ursprünglichen Ausgangskonzentrationen der drei Substanzen in der ursprünglichen Menge an Essigsäure wieder erreicht werden. Nach erneuter Reaktion wird wie oben beschrieben kristallisiert und das Diketon abgetrennt. Der Vorgang der Reaktion und Kristallisation wird insgesamt zehnmal durchgeführt; die eingesetzten Stoffmengen Isosorbid und Katalysator sowie die erhaltenen Stoffmengen an Diketon sind in **Figur 1** und **Tab. 1** zusammengefasst. Aus den Daten ergibt sich, dass der AA-TEMPO-Katalysator 3,5-fach und der HNO₃-Katalysator 2,6-fach wiederverwendet werden kann und zugleich die Gesamtausbeute für Diketon einen Wert (bezogen auf eingesetztes Isosorbid) von 93 % erreicht.

**Tab. 1: Einwaagen an Isosorbid, AA-TEMPO und Salpetersäure sowie Auswaagen an Diketon bei Oxidation von Isosorbid; 10-faches Recycling-Experiment**

| Versuch Nr. | m_{Isosorbid} [g] | m_{AA-Tempo} [g] | m_{HNO3} [g] | m_{Diketon} [g] |
|---|---|---|---|---|
| 1 | 14,64 | 1,50 | 0,47 | 3,77 |
| 2 | 14,00 | -- | -- | 10,19 |
| 3 | 14,70 | -- | -- | 9,89 |
| 4 | 14,66 | -- | -- | 1,12 |
| 5 | 11,17 | 0,70 | -- | 0,86 |
| 6 | -- | -- | 0,47 | 8,56 |
| 7 | 14,17 | 0,30 | -- | 44,57 |
| 8 | 14,72 | -- | -- | 0,00 |
| 9 | 7,77 | 0,75 | 0,24 | 26,66 |
| 10 | 14,77 | -- | 0,24 | 0,00 |
| Summen [g] | 120,60 | 3,25 | 1,42 | 105,63 |
| Summen [mmol] | 825,24 | 16,31 | 22,54 | 743,30 |
| mol% | 100,0 | 2,0 | 2,7 | 92,6 |

## Patentansprüche

1. Verfahren zur Oxidation eines primären oder sekundären Alkohols umfassend aliphatische, cycloaliphatische und aromatische Alkohole, zu einem Aldehyd oder Keton als Kristallinem Reaktionsprodukt, umfassend die folgenden Schritte:
a) Bereitstellen einer Katalysatorzusammensetzung umfassend wenigstens eine nitroxylradikalhaltige Verbindung, wenigstens eine NO-Quelle, wenigstens eine Carbon- oder Mineralsäure oder ein Anhydrid einer Carbon- oder Mineralsäure,
b) Herstellen einer Reaktionsmischung durch Zugabe von wenigstens einem primären oder sekundären Alkohol und einem Sauerstoff umfassenden Gas sowie optional einem oder mehr als einem Lösungsmittel zur Katalysatorenzusammensetzung aus Schritt a) bzw. Schritt e),
c) Inkubieren der Reaktionsmischung aus Schritt b) bei einer Temperatur zwischen 0 und 100 °C oder am Siedepunkt der Lösungsmittels,
d) Gleichzeitig mit oder nachfolgend zu Schritt c) Kristallisation des Reaktionsproduktes, und
e) Rückgewinnen der Katalysatorenzusammensetzung durch Entfernung des kristallisierten Reaktionsproduktes aus der in Schritt d) erhaltenen Reaktionsmischung.

2. Verfahren nach Anspruch 1, wobei die Schritte b) bis e) wenigstens einmal, bevorzugt wenigstens dreimal, wiederholt werden und bei einer jeden Wiederholung von Schritt b) die Katalysatorenzusammensetzung aus Schritt e) verwendet wird.

3. Verfahren nach einem der Ansprüche 1 bis 2, wobei der Alkohol ein mehrwertiger Alkohol ist.

4. Verfahren nach einem der Ansprüche 1 bis 2, wobei der Alkohol aus der Gruppe ausgewählt ist, die aliphatische und lineare ω-Hydroxycarbonsäuren, Zuckeralkohole, bevorzugt dizyklische Zuckeralkohole, und Polyole umfasst.

5. Verfahren nach Anspruch 3, wobei der Alkohol aus der Gruppe ausgewählt ist, die 1,4:3,6-Dianhydro-D-Mannitol, 1,4:3,6-Dianhydro-D-Glucitol (Isosorbit) und 1,4:3,6-Dianhydro-D-Iditol umfasst und bevorzugt 1,4:3,6-Dianhydro-D-Glucitol (Isosorbit) ist.

6. Verfahren nach einem der Ansprüche 1 bis 4, wobei es sich bei der wenigstens einen nitroxylradikalhaltigen Verbindung um eine Verbindung der Formel I oder II handelt, 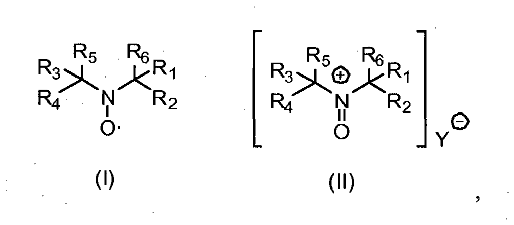 wobei R₁, R₂, R₃, R₄, R₅ und R₆ jeweils unabhängig voneinander aus der Gruppe ausgewählt sind, die (C1-C10)-Alkyl-, (C1-C10)-Alkenyl-, (C1-C10)-Alkoxy-, (C6-C18)-Aryl-, (C7-C19)-Aralkyl-, (C6-C18)-Aryl-(C1-C8)-Alkyl- oder (C3-C18)-Heteroaryl umfasst,
wobei die Substituenten vom Typ R₁, R₂, R₃, R₄, R₅ und R₆ gleich oder verschieden sind und die Substituenten vom Typ R₅ und R₆ zusammen eine (C1-C4)-Alkylenbrücke bilden können, welche gesättigt oder ungesättigt, unsubstituiert oder substituiert sein kann, insbesondere mit einem oder mehreren Substituenten ausgewählt aus der Gruppe umfassend R₁, C1-C8-Amido-, Halogen, Oxy-, Hydroxy-, Amino-, Alkylamino-, Dialkylamino-, Arylamino-, Diarylamino-, Alkylcarbonyloxy-, Arylcarbonyloxy-, Alkylcarbonylamino- und Arylcarbonylaminogruppe,
und wobei Y- ein beliebiges halogenfreies Anion darstellt.

7. Verfahren nach Anspruch 6, wobei es sich bei der wenigstens einen nitroxylradikalhaltigen Verbindung um 2,2,6,6-Tetramethylpiperidin-1-oxyl (TEMPO) und/oder die an Position 4 des Heterocyclus substituierten Derivate des 2,2,6,6-Tetramethylpiperidin-1-oxyls handelt,
wobei die Derivate einen oder mehrere Substituenten ausgewählt aus der Gruppe, die R₇, C₁-C₈-Amido-, Halogen, Oxy-, Hydroxy-, Amino-, Alkylamino-, Dialkylamino-, Arylamino-, Diarylamino-, Alkylcarbonyloxy-, Arylcarbonyloxy-, Alkylcarbonylamino- und Arylcarbonylaminogruppen umfasst, aufweisen,
und wobei R₇ aus der Gruppe ausgewählt ist, die (C₁-C₁₀)-Alkyl-, (C₁-C₁₀)-Alkenyl-, (C₁-C₁₀)-Alkoxy-, (C₆-C₁₈)-Aryl-, (C₇-C₁₉)-Aralkyl-, (C₆-C₁₈)-Aryl-(C₁-C₈)-Alkyl- oder (C₃-C₁₈)-Heteroarylgruppe umfasst.

8. Verfahren nach einem der Ansprüche 1 bis 6, wobei die NO-Quelle aus der Gruppe ausgewählt ist, die Sauerstoffsäuren des Stickstoffes sowie deren Salze, bevorzugt, Salpetersäure und salpetrige Säure, sowie stickoxidhaltige Gase umfasst.

9. Verfahren nach einem der Ansprüche 1 bis 7, wobei die Mineralsäure bzw. ihr Anhdrid aus der Gruppe ausgewählt ist, die H₂CO₃, H₃PO₄, HNO₃, HNO₂, H₂SO₄, H₂SO₃, H₃BO₃ oder deren Anhydride umfasst.

10. Verfahren nach einem der Ansprüche 1 bis 9, wobei Schritt c) und d) getrennt durchgeführt werden.

11. Verfahren nach einem der Ansprüche 1 bis 9, wobei Schritt c) und d) gleichzeitig durchgeführt werden.

12. Verfahren nach einem der Ansprüche 1 bis 11, wobei Schritt c) bei einer Temperatur von unter 70°C durchgeführt wird.

13. Verfahren nach Anspruch 12, wobei Schritt c) bei einer Temperatur von unter 50 °C, bevorzugt unter 45 °C, durchgeführt wird.

14. Verfahren nach Anspruch 13, wobei Schritt c) bei einer Temperatur von 0 bis 100, bevorzugter 0 bis 70; 10 bis 50; 10 bis 45; noch bevorzugter 10 bis 45; 15 bis 40 °C, 20 bis 40 °C, am bevorzugtesten bei 20 bis 35 °C ausgeführt wird.

## Claims

1. Method for oxidizing a primary or secondary alcohol comprising aliphatic, cycloaliphatic and aromatic alcohols to an aldehyde or ketone as crystalline reaction product, comprising the following steps:
a) providing a catalyst composition comprising at least one nitroxyl radical-containing compound, at least one NO source, at least one carboxylic or mineral acid or an anhydride of a carboxylic or mineral acid,
b) preparing a reaction mixture by adding at least one primary or secondary alcohol and a gas comprising oxygen and optionally also one or more solvents to the catalyst composition from step a) and step e),
c) incubating the reaction mixture from step b) at a temperature between 0 and 100°C or at the boiling point of the solvent,
d) crystallizing the reaction product at the same time as or after step c), and
e) recovering the catalyst composition by removing the crystallized reaction product from the reaction mixture obtained in step d).

2. Method according to Claim 1, wherein the steps b) to e) are repeated at least once, preferably at least three times and with each repetition of step b) the catalyst composition from step e) is used.

3. Method according to either of Claims 1 and 2, wherein the alcohol is a polyhydric alcohol.

4. Method according to either of Claims 1 and 2, wherein the alcohol is selected from the group comprising aliphatic and linear □-hydroxycarboxylic acids, sugar alcohols, preferably bicyclic sugar alcohols, and polyols.

5. Method according to Claim 3, wherein the alcohol is selected from the group comprising 1,4:3,6-dianhydro-D-mannitol, 1,4:3,6-dianhydro-D-glucitol (isosorbitol) and 1,4:3,6-dianhydro-D-iditol and is preferably 1,4:3,6-dianhydro-D-glucitol (isosorbitol).

6. Method according to any of Claims 1 to 4, wherein the compound containing at least one nitroxyl radical is a compound of the formula I or II, 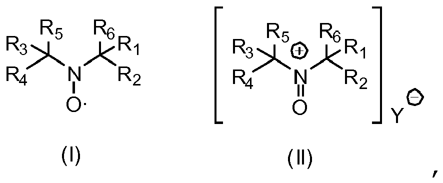 where R₁, R₂, R₃, R₄, R₅ and R₆ are each independently selected from the group comprising (C1-C10)-alkyl, (C1-C10)-alkenyl, (C1-C10)-alkoxy, (C6-C18)-aryl, (C7-C19)-aralkyl, (C6-C18)-aryl-(C1-C8)-alkyl and (C3-C18)-heteroaryl,
where the substituents of the type R₁, R₂, R₃, R₄, R₅ and R₆ are identical or different and the substituents of the type R₅ and R₆ can together form a (C1-C4)-alkylene bridge, which may be saturated or unsaturated, unsubstituted or substituted, particularly having one or more substituents selected from the group comprising R₁, C1-C8-amido, halogen, oxy, hydroxyl, amino, alkylamino, dialkylamino, arylamino, diarylamino, alkylcarbonyloxy, arylcarbonyloxy, alkylcarbonylamino and arylcarbonylamino groups,
and where Y⁻ is any halogen-free anion.

7. Method according to Claim 6, wherein the compound containing at least one nitroxyl radical is 2,2,6,6-tetramethylpiperidin-1-oxyl (TEMPO) and/or the derivatives of 2,2,6,6-tetramethylpiperidin-1-oxyl substituted at position 4 of the heterocycle,
wherein the derivatives have one or more substituents selected from the group comprising R₇, C₁-C₈-amido, halogen, oxy, hydroxyl, amino, alkylamino, dialkylamino, arylamino, diarylamino, alkylcarbonyloxy, arylcarbonyloxy, alkylcarbonylamino and arylcarbonylamino groups,
and where R₇ is selected from the group comprising (C₁-C₁₀)-alkyl, (C₁-C₁₀) -alkenyl, (C₁-C₁₀) -alkoxy, (C₆-C₁₀)-aryl, (C₇-C₁₉) -aralkyl, (C₆-C₁₈) -aryl- (C₁-C₈) -alkyl and (C₃-C₁₈)-heteroaryl groups.

8. Method according to any of Claims 1 to 6, where the NO source is selected from the group comprising oxygen acids of nitrogen and also salts thereof, preferably nitric acid and nitrous acid, and also nitrogen oxide-containing gases.

9. Method according to any of Claims 1 to 7, wherein the mineral acid or the anhydride thereof is selected from the group comprising H₂CO₃, H₃PO₄, HNO₃, HNO₂, H₂SO₄, H₂SO₃, H₃BO₃ or anhydrides thereof.

10. Method according to any of Claims 1 to 9, wherein steps c) and d) are carried out separately.

11. Method according to any of Claims 1 to 9, wherein steps c) and d) are carried out simultaneously.

12. Method according to any of Claims 1 to 11, wherein steps c) is carried out at a temperature below 70°C.

13. Method according to Claim 12, wherein step c) is carried out at a temperature below 50°C, preferably below 45°C.

14. Method according to Claim 13, wherein step c) is conducted at a temperature of 0 to 100, preferably 0 to 70; 10 to 50; 10 to 45; more preferably 10 to 45; 15 to 40°C, 20 to 40°C, most preferably at 20 to 35°C.

## Revendications

1. Procédé pour l'oxydation d'un alcool primaire ou secondaire comprenant des alcools aliphatiques, cycloaliphatiques et aromatiques en un aldéhyde ou une cétone comme produit de réaction cristallin, comprenant les étapes suivantes :
a) préparation d'une composition de catalyseur comprenant au moins un composé contenant des radicaux nitroxyle, au moins une source de NO, au moins un acide carboxylique ou minéral ou un anhydride d'un acide carboxylique ou minéral,
b) préparation d'un mélange réactionnel par addition d'au moins un alcool primaire ou secondaire et d'un gaz contenant de l'oxygène ainsi qu'éventuellement d'un ou plus d'un solvant à la composition de catalyseur de l'étape a) ou de l'étape e),
c) incubation du mélange réactionnel de l'étape b) à une température entre 0 et 100°C ou au point d'ébullition du solvant,
d) cristallisation du produit de réaction simultanément avec l'étape c) ou consécutivement à celle-ci et
e) récupération de la composition de catalyseur par élimination du produit de réaction cristallisé du mélange réactionnel obtenu dans l'étape d).

2. Procédé selon la revendication 1, les étapes b) à e) étant répétées au moins une fois, de préférence au moins trois fois, et la composition de catalyseur de l'étape e) étant utilisée lors de chaque répétition de l'étape b).

3. Procédé selon l'une quelconque des revendications 1 à 2, l'alcool étant un alcool polyvalent.

4. Procédé selon l'une quelconque des revendications 1 à 2, l'alcool étant choisi dans le groupe comprenant les acides ω-hydroxycarboxyliques aliphatiques et linéaires, les alcools de sucre, de préférence les alcools de sucre dicycliques, et les polyols.

5. Procédé selon la revendication 3, l'alcool étant choisi dans le groupe comprenant le 1,4:3,6-dianhydro-D-mannitol, le 1,4:3,6-dianhydro-D-glucitol (isosorbitol) et le 1,4:3,6-dianhydro-D-iditol et étant de préférence le 1,4:3,6-dianhydro-D-glucitol (isosorbitol).

6. Procédé selon l'une quelconque des revendications 1 à 4, où il s'agit, pour ledit au moins un composé contenant des radicaux nitroxyle, d'un composé de formule I ou II, 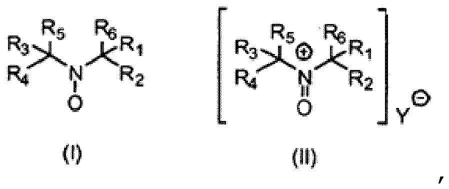 R₁, R₂, R₃, R₄, R₅ et R₆ étant choisis, à chaque fois indépendamment les uns des autres, dans le groupe comprenant (C₁-C₁₀)-alkyle, (C₁-C₁₀)-alcényle, (C₁-C₁₀)-alcoxy, (C₆-C₁₈) -aryle, (C₇-C₁₉) -aralkyle, (C₆-C₁₈) -aryl-(C₁-C₈)-alkyle ou (C₃-C₁₈)-hétéroaryle,
les substituants du type R₁, R₂, R₃, R₄, R₅ et R₆ étant identiques ou différents et les substituant du type R₅ et R₆ pouvant former ensemble un pont (C₁-C₄)-alkylène, qui peut être saturé ou insaturé, non substitué ou substitué, en particulier par un ou plusieurs substituants choisis dans le groupe comprenant R₁, un groupe C₁-C₈-amido, halogène, oxy, hydroxy, amino, alkylamino, dialkylamino, arylamino, diarylamino, alkylcarbonyloxy, arylcarbonyloxy, alkylcarbonylamino et arylcarbonylamino,
et Y représentant un anion quelconque exempt d'halogène.

7. Procédé selon la revendication 6, où il s'agit, pour ledit au moins un composé contenant des radicaux nitroxyle, de 2,2,6,6-tétraméthylpipéridin-1-oxyle (TEMPO) et/ou des dérivés substitués en position 4 de l'hétérocycle du 2,2,6,6-tétraméthylpipéridin-1-oxyle, les dérivés présentant un ou plusieurs substituants choisis dans le groupe comprenant R₇, un groupe C₁-C₈-amido, halogène, oxy, hydroxy, amino, alkylamino, dialkylamino, arylamino, diarylamino, alkylcarbonyloxy, arylcarbonyloxy, alkylcarbonylamino et arylcarbonylamino,
R₇ étant choisi dans le groupe comprenant le groupe (C₁-C₁₀)-alkyle, (C₁-C₁₀)-alcényle, (C₁-C₁₀)-alcoxy, (C₆-C₁₈)-aryle, (C₇-C₁₉)-aralkyle, (C₆-C₁₈) -aryl-(C₁-C₈)-alkyle ou (C₃-C₁₈)-hétéroaryle.

8. Procédé selon l'une quelconque des revendications 1 à 6, la source de NO étant choisie dans le groupe comprenant les acides oxygénés de l'azote ainsi que leurs sels, de préférence l'acide nitrique et l'acide nitreux, ainsi que les gaz contenant de l'oxyde d'azote.

9. Procédé selon l'une quelconque des revendications 1 à 7, l'acide minéral ou son anhydride étant choisi dans le groupe comprenant H₂CO₃, H₃PO₄, HNO₃, HNO₂, H₂SO₄, H₂SO₃, H₃BO₃ ou leurs anhydrides.

10. Procédé selon l'une quelconque des revendications 1 à 9, l'étape c) et l'étape d) étant réalisées séparément.

11. Procédé selon l'une quelconque des revendications 1 à 9, l'étape c) et l'étape d) étant réalisées simultanément.

12. Procédé selon l'une quelconque des revendications 1 à 11, l'étape c) étant réalisée à une température inférieure à 70°C.

13. Procédé selon la revendication 12, l'étape c) étant réalisée à une température inférieure à 50°C, de préférence inférieure à 45°C.

14. Procédé selon la revendication 13, l'étape c) étant réalisée à une température de 0 à 100, de préférence 0 à 70 ; 10 à 50 ; 10 à 45 ; plus préférablement de 10 à 45 ; 15 à 40°C, 20 à 40°C, le plus préférablement de 20 à 35°C.
